# EUROPÄISCHE PATENTSCHRIFT

(11) **EP 3 021 885 B1**
(45) Veröffentlichungstag und Bekanntmachung des Hinweises auf die Patenterteilung: **22.04.2020**
(21) Anmeldenummer: 14742173.9
(22) Anmeldetag: 14.07.2014
(51) Int. Cl.: A61M 1/06

(54) **BRUSTPUMPENEINHEIT**
BREAST PUMP UNIT
UNITÉ DE POMPE TIRE-LAIT

(30) Priorität: 16.07.2013 CH 12672013
(43) Veröffentlichungstag der Anmeldung: 25.05.2016
(73) Patentinhaber: Medela Holding AG, 6340 Baar (CH)
(72) Erfinder: FELBER, Armin, CH-6003 Luzern (CH)
(74) Vertreter: Clerc, Natalia
(86) Internationale Anmeldenummer: PCT/EP2014/065024
(87) Internationale Veröffentlichungsnummer: WO 2015/007679

(56) Entgegenhaltungen:
- WO-A1-2011/144984
- WO-A1-2013/049944
- WO-A2-2011/037841
- US-A1- 2005 137 539
- US-A1- 2006 211 335
- US-A1- 2011 106 027

## Beschreibung

### TECHNISCHES GEBIET

Die vorliegende Erfindung betrifft eine Brustpumpeneinheit zum Abpumpen menschlicher Muttermilch.

### STAND DER TECHNIK

Im Stand der Technik sind manuell wie auch motorisch betriebene Brustpumpen bekannt. Die mit einem Elektromotor versehenen Brustpumpen können an ein Stromversorgungsnetz angeschlossen oder batteriebetrieben sein. Diese Brustpumpen umfassen eine Vakuumpumpe sowie eine oder zwei mit der Vakuumpumpe verbundene Brusthauben zur Anlage an die Mutterbrust bzw. an die Mutterbrüste. Die Verbindung kann direkt sein, d.h. die Vakuumpumpe ist an der Brusthaube selber angeordnet, oder Vakuumpumpe und Brusthaube sind über einen Saugschlauch, auch Vakuumschlauch genannt, miteinander verbunden. Mittels der Vakuumpumpe lässt sich ein Unterdruck in der Brusthaube erzeugen, wodurch Milch aus der Mutterbrust gesaugt wird.

Die Brusthaube ist mit einem Milchauffangbehälter verbunden, in welchem die abgepumpte Milch gesammelt wird. Milchauffangbehälter können Beutel oder Flaschen sein, welche luftdicht mit einem entsprechenden Ausgang der Brusthaube verbindbar sind. Eine bekannte Brustpumpeneinheit ist beispielsweise in WO 01/47577 beschrieben.

Um den erzeugten Unterdruck zu begrenzen, weisen einige der motorisch betriebenen und elektronisch gesteuerten Vakuumpumpen einen Drucksensor auf. Beispiele hierfür sind in US 6 383 163, US 8 137 305 und US 2008/0009815 offenbart. Diese Drucksensoren messen jeweils den Druck im pumpennahen Bereich der Saugleitung bzw. beim Sauganschluss der Vakuumpumpe selber. In US 2007/0060873 ist eine Brusthaube mit darauf aufgesetzter Vakuumpumpe offenbart, wobei die Vakuumpumpe mit einer Medientrennmembran und einem Druckanzeiger ausgerüstet ist.

In WO 2013/049944 wird vorgeschlagen, den Saugschlauch auch als Milchleitung zu verwenden. Eine Medientrennmembran zwischen pumpenseitigem Ende des Saugschlauchs und Vakuumpumpe schützt dabei die Vakuumpumpe vor einer Verunreinigung durch Milch. Diese Medientrennmembran ist mit einem Drucksensor versehen, um den Druck in diesem Bereich festzustellen.

In WO 2011/037841 wird vorgeschlagen, einen Drucksensor in der Brusthaube selber anzuordnen und über eine elektronische Leitung mit einer Steuerung oder Controller der Vakuumpumpe zu verbinden. Brusthauben sind jedoch Einwegprodukte oder beim Mehrfachgebrauch zumindest nicht für den längeren Gebrauch ausgelegt. Sie müssen deshalb kostengünstig sein. Im Falle eines Mehrfachgebrauchs müssen sie auch einfach zu reinigen sein, so dass Drucksensoren innerhalb oder an der Brusthaube störend sind.

WO 2011/144984 offenbart eine Vorrichtung, welche zum direkten Stillen des Säuglings bestimmt ist. Auch diese Vorrichtung umfasst eine Vakuumpumpe zur Erzeugung eines Unterdrucks und eine Brusthaube zur Auflage auf eine menschliche Mutterbrust. Die Brusthaube endet hier in einem Milchspeicher, welcher über eine Leitung mit einem Sauger für den Säugling verbunden ist. Zuerst wird Milch mittels der Vakuumpumpe abgepumpt und im Milchspeicher gesammelt. Anschliessend wird die Vakuumpumpe gestoppt. Fängt der Säugling nun an zu saugen, so stellt ein Vakuumsensor einen Trink-Unterdruck fest und er lässt über eine Steuereinheit ein Ventil öffnen, um den Unterdruck im Milchspeicher auf Atmosphärendruck anzuheben. Anschliessend kann der Säugling saugen. Solange der Trink-Unterdruck detektiert wird, bleibt die Vakuumpumpe inaktiv.

US 2006/0211335 offenbart eine Brusthaube mit flexiblen Kissen, welche mittels einer Vakuumpumpe mit einem Unter- bzw. Überdruck versehen werden können. Druckänderungen lassen sich in der Vakuumleitung zwischen Kissen und Vakuumpumpe messen.

### DARSTELLUNG DER ERFINDUNG

Es ist eine Aufgabe der Erfindung, die Druckmessung beim Abpumpen von menschlicher Muttermilch zu optimieren.

Diese Aufgabe löst eine Brustpumpeneinheit mit den Merkmalen des Anspruchs 1.

Die erfindungsgemässe Brustpumpeneinheit zum Abpumpen von menschlicher Muttermilch weist eine Brusthaube zum Anlegen auf eine menschliche Mutterbrust, eine Vakuumpumpe zur Erzeugung eines Unterdrucks und eine Saugleitung zur Verbindung der Brusthaube mit der Vakuumpumpe und zur Übertragung des von der Vakuumpumpe erzeugten Unterdrucks in die Brusthaube auf. Ferner ist ein Drucksensor vorhanden. Erfindungsgemäss ist zusätzlich eine Messleitung vorhanden, wobei sich die Messleitung von der Brusthaube bis zum Drucksensor erstreckt. Vorzugsweise endet sie am Drucksensor.

Dank der erfindungsgemässen Einheit lässt sich der Druck am Ort der gewünschten Wirkung der Brustpumpe messen, nämlich im Bereich nahe bei der Mutterbrust. Trotzdem müssen die Brusthauben nicht mit kostenintensiven Sensoren ausgerüstet werden.

Die erfindungsgemässe Anordnung mit der separaten, insbesondere luftgefüllten Messleitung lässt sich in allen bekannten Brustpumpeneinheiten verwenden. Sie ist jedoch insbesondere in Brustpumpeneinheiten vorteilhaft, in welchen der Saugschlauch auch als Milchleitung verwendet wird. Da im Saugschlauch somit eine Flüssigkeitssäule vorhanden ist, welche sich je nach Lage des Saugschlauchs ändert, kann dies zu einer verfälschten Druckmessung führen. Die Verwendung einer separaten, luftgefüllten Messleitung, vorzugsweise eines Messschlauchs, garantiert eine genaue Messung des Drucks, unabhängig von der Lage der Vakuumpumpe, der Länge des Saugschlauchs und der Lage des Saugschlauchs sowie des Messschlauchs.

In einer bevorzugten Ausführungsform ist der Drucksensor in der Brustpumpe angeordnet. Die Brustpumpe weist ein Gehäuse auf, in welchem die Vakuumpumpe, eine Steuerelektronik zur Steuerung der Vakuumpumpe, Bedienungsmittel zur Bedienung der Steuerelektronik, allenfalls Anzeigemittel und auch der Drucksensor angeordnet sind.

In einer einfachsten Ausführungsform dient der Drucksensor nur als Sicherheitselement, damit kein zu hoher Unterdruck angelegt wird. Alternativ oder zusätzlich kann das Sicherheitselement zur Feststellung dienen, ob überhaupt ein Unterdruck in der Brusthaube vorliegt. Falls zum Beispiel die Brusthaube nicht dichtend genug auf die Mutterbrust aufgelegt wird, kann ein Warnsignal oder ein optisches Signal ertönen.

In einer bevorzugten Ausführungsform ist die elektronische Steuerung zur Steuerung der Vakuumpumpe mit dem Drucksensor verbunden. Dadurch lässt sich die Vakuumpumpe nach Massgabe der gemessenen Druckwerte innerhalb der Brusthaube steuern und der an die Mutterbrust angelegte Unterdruck wird optimiert. In einer bevorzugten Ausführungsform übernimmt der Drucksensor auch eine oder beide der oben genannten Sicherheitsfunktionen.

Die Messleitung und die Saugleitung sind vorzugsweise Schläuche, insbesondere flexible Schläuche. Sie lassen sich beispielsweise aus Silikon fertigen. In einer bevorzugten Ausführungsform sind die Messleitung und die Saugleitung gemeinsam als doppellumiger Schlauch ausgebildet. Dies erleichtert das Einstecken und die generelle Handhabung der Brustpumpeneinheit.

In einer bevorzugten Ausführungsform weist die Saugleitung ein erstes brusthaubenseitiges Ende und die Messleitung weist ein zweites brusthaubenseitiges Ende auf, wobei diese zwei Enden getrennt voneinander verlaufen. Dies erleichtert das Einstecken der Schläuche sowie die Ausstattung der Brusthaube mit einem nachfolgend beschriebenen Schutzelement bzw. Medientrenneinrichtung.

Der Unterdruck wird üblicherweise in einer Saugkammer der Brusthaube erzeugt, wobei der Unterdruck in dieser Saugkammer messbar ist. In einer Ausführungsform mündet die Messleitung in diese Saugkammer. Dadurch wird tatsächlich vor Ort gemessen, d.h. dort, wo sich auch die Brustwarze der Mutterbrust befindet.

Vorzugsweise ist ein Schutzelement vorhanden, welches die Messleitung von einem mit abgepumpter Muttermilch beaufschlagbaren Innenraum der Brusthaube trennt. Dadurch werden die Messleitung und somit auch der Drucksensor vor Verunreinigung mit Muttermilch geschützt. Das Schutzelement ist flüssigkeitsundurchlässig. Es kann ein luftdurchlässiges Filter oder ein luft- und flüssigkeitsundurchlässiges Element sein.

Vorzugsweise ist eine Medientrenneinrichtung vorhanden, welche die Messleitung von einem Innenraum der Brusthaube trennt, welche jedoch Druckveränderungen im Innenraum zur Messleitung übermittelt. Vorzugsweise ist die Medientrenneinrichtung eine flexible, fluidundurchlässige Membran, wie sie auch in anderen Bereichen von Brustpumpeneinheiten, z.B. beim Vakuumanschluss der Brustpumpe und auch beim brusthaubenseitigen Anschluss des Saugschlauchs bereits eingesetzt ist. Vorzugsweise ist der Innenraum die oben genannte Saugkammer. Vorzugsweise ist die Medientrenneinrichtung das oben genannte Schutzelement.

Damit die Brusthaube einfach gereinigt bzw. nach Gebrauch entsorgt werden kann, ist die Messleitung vorzugsweise mehrmals lösbar mit der Brusthaube verbindbar. Dadurch kann die Messleitung, insbesondere jedoch der Drucksensor, mehrfach verwendet werden. Alternativ ist die Messleitung nicht zerstörungsfrei mit der Brusthaube verbunden, jedoch einfach vom Drucksensor lösbar, ohne diesen zu beinträchtigen.

Eine in der erfindungsgemässen Brustpumpeneinheit verwendete Brusthaube weist eine erste Aufnahme zur Aufnahme einer Saugleitung und eine zweite Aufnahme zur Aufnahme einer Messleitung auf.

Bei geeigneter Ausbildung eines doppellumigen Saug- und Messschlauchs lässt sich dieser in eine einzige Öffnung der Brusthaube einstecken. Vorzugsweise ist die erste Aufnahme eine Saugöffnung zur Aufnahme eines Saugschlauchs und die zweite Aufnahme eine davon separate Messöffnung zur Aufnahme einer Messleitung. Vorzugsweise ist die zweite Aufnahme mit einer Medientrennvorrichtung versehen.

Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:
- Figur 1: eine schematische Darstellung einer erfindungsgemässen Brustpumpeneinheit in einer ersten Ausführungsform;
- Figur 2: eine schematische Darstellung einer erfindungsgemässen Brustpumpeneinheit in einer zweiten Ausführungsform;
- Figur 3: eine schematische Darstellung einer erfindungsgemässen Brustpumpeneinheit in einer dritten Ausführungsform;
- Figur 4: einen vergrösserten Ausschnitt gemäss Figur 3;
- Figur 5: eine schematische Darstellung einer erfindungsgemässen Brustpumpeneinheit in einer vierten Ausführungsform und
- Figur 6: einen vergrösserten Ausschnitt gemäss Figur 5.

### BESCHREIBUNG BEVORZUGTER AUSFÜHRUNGSFORMEN

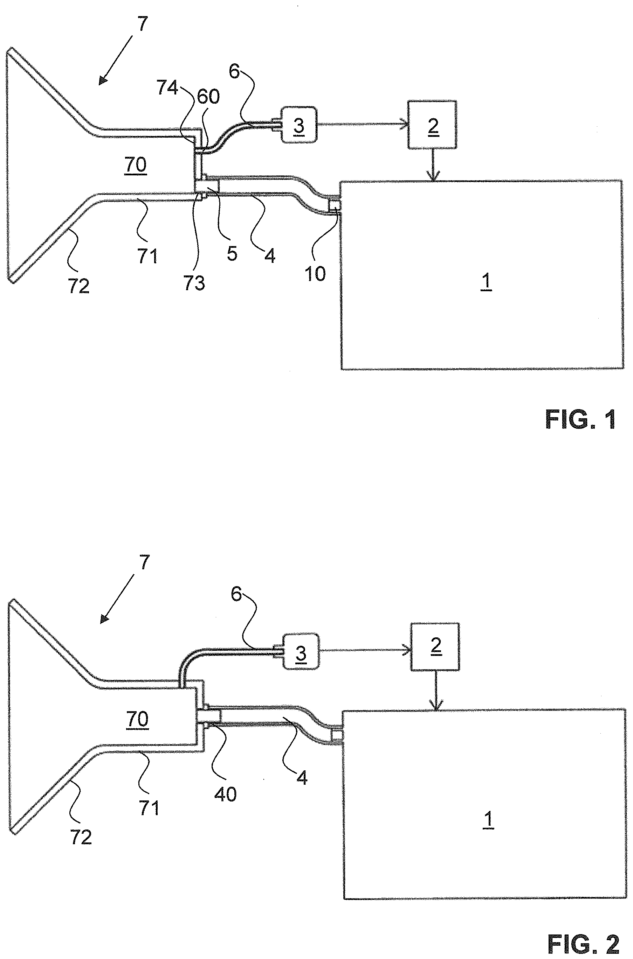

Figur 1 zeigt ein erstes Beispiel einer erfindungsgemässen Brustpumpeneinheit. Eine Vakuumpumpe 1 weist einen Sauganschluss 10 auf. Mit diesem Sauganschluss 10 ist eine Saugleitung, hier ein Saugschlauch 4, verbunden. Der Saugschlauch 4 ist vorzugsweise ein üblicher Silikonschlauch, wie er für den Einsatz in Brustpumpeneinheiten üblicherweise verwendet wird.

Die Brustpumpeneinheit umfasst ferner eine oder zwei Brusthauben 7 zur Auflage auf die menschliche Mutterbrust. Als Brusthaube 7 eignen sich die im Stand der Technik bekannten Arten. Die dargestellte Form und Ausgestaltung ist lediglich exemplarisch.

Die hier dargestellte Brusthaube 7 weist einen Stutzen 71 und einen daran angeformten, sich nach aussen erweiternden Trichter 72 auf. In letzteren wird die Mutterbrust aufgenommen. In ersteren kann je nach Ausgestaltung der Brusthaube die Brustwarze der Mutterbrust hineinragen. Die Brusthaube 7 weist einen Innenraum 70 und eine von diesem Innenraum 70 nach aussen führende erste Öffnung 73 auf. In diese erste Öffnung 73 lässt sich der oben genannte Saugschlauch 4 mit seinem ersten brusthaubenseitigen Ende 40 einstecken oder anderweitig mit einer über der ersten Öffnung 73 ausgebildeten Aufnahme verbinden. In diesen Figuren ist ein Anschlussstück 5 zum Einstecken vorgesehen.

Ein Milchsammelbehälter ist nicht dargestellt. Er kann auf bekannte Art und Weise direkt an der Brusthaube montierbar sein. Die Saugleitung kann jedoch auch gleichzeitig als Milchleitung dienen, so dass der Milchsammelbehälter über eine nicht dargestellte, aus dem Element 1 hinausführende Leitung befüllt wird. Diese Kombination von Saugleitung und Milchleitung ist in WO 2013/049944 offenbart.

Die Brusthaube 7 weist eine zweite Öffnung 74 auf, welche ebenfalls vom Innenraum 70 nach aussen führt. Von dieser Aufnahme oder Öffnung 74 führt eine Messleitung 6 zu einem Drucksensor 3. Die Messleitung 6 ist vorzugsweise ebenfalls ein Schlauch, beispielsweise ein Silikonschlauch. Vorzugsweise weist er jedoch einen kleineren Durchmesser auf als der Saugschlauch 4. Der Messschlauch 6 ist vorzugsweise mit Luft gefüllt.

In diesem Beispiel ist ein zweites brusthaubenseitiges Ende 60 des Messschlauchs 6 in die zweite Öffnung 74 eingesteckt. Er kann auch auf andere Art und Weise mit der Brusthaube 7 verbunden sein. Vorzugsweise ist die Verbindung zwischen Messleitung 6 und Brusthaube 7 so ausgebildet, dass sie gelöst und wieder zusammen gebracht werden kann. Sind Messleitung 6 und Brusthaube 7 nicht zerstörungsfrei voneinander lösbar, so ist vorzugsweise die Verbindung zwischen Messleitung 6 und Sensor 3 lösbar, ohne den Sensor 3 bzw. die sensorseitige Steckverbindung zu beschädigen.

Der Saugschlauch 4 und der Messschlauch 6 sind in diesen Beispielen als voneinander getrennt verlaufende Schläuche dargestellt. Es lässt sich jedoch auch ein doppellumiger Schlauch verwenden.

Als Drucksensor 3 eignen sich bekannte Sensoren, beispielsweise piezoelektrische Sensoren. Der Drucksensor ist mit einer elektronischen Steuereinheit 2 der Vakuumpumpe verbunden. Die Leitungen zwischen Steuerung 2 und Drucksensor 3 bzw. zwischen Steuerung 2 und Vakuumpumpe 1 sind hier mit Pfeilen versehen.

Vakuumpumpe 1, Steuerung 2 und Drucksensor 3 sind vorzugsweise gemeinsam mit manuellen Bedienungsmitteln sowie einer allfälligen Anzeige bzw. einem Bildschirm in einem gemeinsamen Gehäuse angeordnet. Das Gehäuse ist hier nicht dargestellt. Die Brusthaube 7 ist dabei getrennt von diesem Gehäuse, wobei die Saugleitung 4 und die Messleitung 6 vom Gehäuse zur Brusthaube 7 führen.

Der Drucksensor 3 misst nun unabhängig von der Lage der Milchleitung und der Lage der Saugleitung 4 den Druck im Innenraum 70 der Brusthaube, also dort, wo über die Vakuumpumpe ein Unterdruck angelegt wird, und dort, wo sich auch die Brustwarze befindet. Das gemessene Signal wird der Steuerung 2 übermittelt, welche je nach Ausgestaltung der Steuerung eine oder mehrere der folgenden Aktionen auslöst:
- Anzeige des Druckwerts auf einem hier nicht dargestellten Bildschirm,
- optische Anzeige oder akustisches Signal bei Unterschreiten oder Überschreiten eines vorgegebenen Minimal- bzw. Maximalwertes,
- automatische Anpassung des von der Vakuumpumpe erzeugten Unterdrucks am Sauganschluss 10,
- Abschalten der Vakuumpumpe im Falle eines Überschreitens eines Maximalwertes, d.h. wenn der angelegte Unterdruck in absoluten Werten zu hoch wird oder, anders gesagt, zu stark gesaugt wird.

Im Beispiel gemäss Figur 1 ist die erste und zweite Öffnung 73, 74 auf derselben Seite der Brusthaube 7 angeordnet; hier an der brustfernen Stirnseite des Stutzens 71.

In der Variante gemäss Figur 2 ist die erste Öffnung 73 nach wie vor in der brustfernen Stirnseite des Stutzens 71 angeordnet. Die zweite Öffnung 74 befindet sich in einem 90° Winkel dazu am Umfang des Stutzens 71.

In den Beispielen gemäss den Figuren 1 und 2 führt die Messleitung 6 direkt und ohne Zwischenelemente in den Innenraum 70. Der Innenraum 70 kann jedoch beispielsweise mit einem Liner versehen sein.

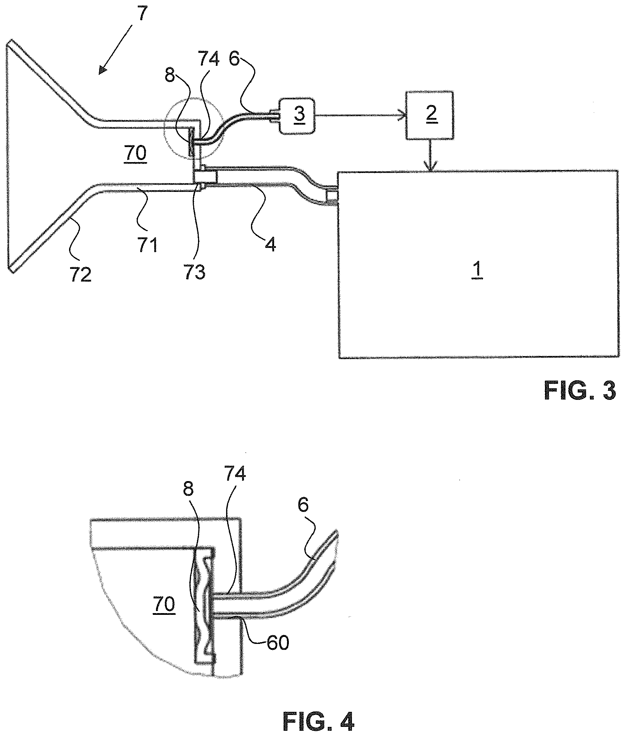

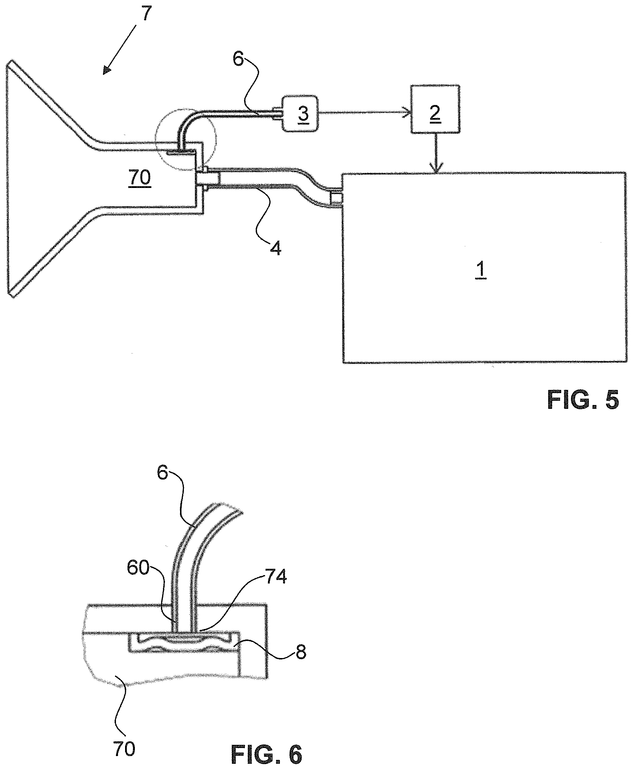

Die Ausführungsformen gemäss den Figuren 3 bis 6 entsprechen den oben beschriebenen. Sie weisen jedoch ein Schutzelement im Bereich der zweiten Öffnung 74 in Form einer Medientrennmembran 8 auf. Sie ist flüssigkeitsdicht ausgebildet und vorzugsweise auch luftdicht. Sie ist vorzugsweise aus Silikon oder einem anderen flexiblen Material gefertigt.

Die Medientrennmembran 8 ist in diesen Beispielen im Innenraum 70 auf der Innenseite des Stutzens 71 befestigt. Sie kann beispielweise in einem Gehäuse angeordnet sein oder einfach an die Wandung des Stutzens 71 angeklebt oder mit diesem verschweisst sein. Sie kann aber auch herausnehmbar sein, um beispielsweise gereinigt zu werden.

Die Medientrennmembran 8 ist vorzugsweise flach und rund ausgebildet. Sie weist kreisrunde Erhebungen und Vertiefungen auf, um sich bei Veränderung des Unterdrucks zu bewegen. Dadurch überträgt sie Druckveränderungen im Innenraum 70 zur Messleitung 6 und somit zum Drucksensor 3. Gleichzeitig verhindert sie, dass abgepumpte Milch und Bakterien oder andere Verunreinigungen in die Messleitung gelangen können.

Die Medientrennmembran kann auch auf der Aussenseite der Brusthaube oder auf dem zweiten brusthaubenseitigen Ende 60 der Messleitung 6 angeordnet sein.

Die erfindungsgemässe Brustpumpeneinheit ermöglicht auf kostengünstige Weise eine relativ genaue Messung des an die Mutterbrust angelegten Unterdrucks.

**BEZUGSZEICHENLISTE**

| | | | |
|---|---|---|---|
| 1 | Vakuumpumpe | | Ende |
| 10 | Sauganschluss | 7 | Brusthaube |
| 2 | elektronische Steuerung | 70 | Innenraum |
| 3 | Drucksensor | 71 | Stutzen |
| 4 | Saugschlauch | 72 | Trichter |
| 40 | erstes brusthaubenseitiges Ende | 73 | erste Öffnung |
| 5 | Anschlussstück | 74 | zweite Öffnung |
| 6 | Messschlauch | 8 | Medientrennmembran |
| 60 | zweites brusthaubenseitiges | | |

## Patentansprüche

1. Brustpumpeneinheit zum Abpumpen von menschlicher Muttermilch, wobei die Brustpumpeneinheit eine Brusthaube (7) zum Anlegen auf eine menschliche Mutterbrust mit einem Innenraum (70) zur Aufnahme der Mutterbrust, eine Vakuumpumpe (1) zur Erzeugung eines Unterdrucks und eine Saugleitung (4) aufweist, wobei die Saugleitung (4) die Brusthaube (7) mit der Vakuumpumpe (1) verbindet und wobei die Saugleitung (4) den von der Vakuumpumpe (1) erzeugten Unterdruck in den Innenraum (70) der Brusthaube (7) überträgt, wobei die Brustpumpeneinheit ferner einen Drucksensor (3) aufweist, **dadurch gekennzeichnet, dass** zusätzlich eine Messleitung (6) vorhanden ist und dass sich die Messleitung (6) von der Brusthaube (7) bis zum Drucksensor (3) erstreckt und der Bestimmung eines Drucks im Innenraum (70) dient.

2. Brustpumpeneinheit nach Anspruch 1, wobei sie eine elektronische Steuerung (2) zur Steuerung der Vakuumpumpe (1) aufweist und wobei der Drucksensor (3) mit der elektronischen Steuerung (2) verbunden ist.

3. Brustpumpeneinheit nach einem der Ansprüche 1 oder 2, wobei die Messleitung (6) und die Saugleitung (4) Schläuche sind.

4. Brustpumpeneinheit nach einem der Ansprüche 1 bis 3, wobei die Messleitung (6) und die Saugleitung (4) gemeinsam als doppellumiger Schlauch ausgebildet sind.

5. Brustpumpeneinheit nach einem der Ansprüche 1 bis 4, wobei die Saugleitung (4) ein erstes brusthaubenseitiges Ende (40) und die Messleitung (6) ein zweites brusthaubenseitiges Ende (60) aufweist und wobei diese zwei Enden (40, 60) getrennt voneinander verlaufen.

6. Brustpumpeneinheit nach einem der Ansprüche 1 bis 5, wobei der Unterdruck in einer Saugkammer (70) der Brusthaube (7) erzeugbar ist und wobei mittels der Messleitung der Unterdruck in der Saugkammer messbar ist.

7. Brustpumpeneinheit nach Anspruch 6, wobei die Messleitung (6) in diese Saugkammer (70) mündet.

8. Brustpumpeneinheit nach einem der Ansprüche 1 bis 6, wobei ein Schutzelement (8) vorhanden ist, welches die Messleitung (6) vom mit abgepumpter Muttermilch beaufschlagbaren Innenraum (70) der Brusthaube (7) trennt.

9. Brustpumpeneinheit nach einem der Ansprüche 1 bis 6, wobei eine Medientrenneinrichtung (8) vorhanden ist, welche die Messleitung (6) vom Innenraum (70) der Brusthaube (7) trennt.

10. Brustpumpeneinheit nach den Ansprüchen 6 und 9, wobei der Innenraum (70) die Saugkammer ist.

11. Brustpumpeneinheit nach den Ansprüchen 8 und 9, wobei das Schutzelement die Medientrenneinrichtung (8) ist.

12. Brustpumpeneinheit nach einem der Ansprüche 9 bis 11, wobei die Medientrenneinrichtung eine flexible, fluidundurchlässige Membran (8) ist.

13. Brustpumpeneinheit nach einem der Ansprüche 1 bis 12, wobei die Messleitung (6) mehrmals lösbar mit der Brusthaube (7) verbindbar ist.

14. Brustpumpeneinheit nach einem der Ansprüche 1 bis 13, wobei die Messleitung (6) beim Drucksensor (3) endet.

## Claims

1. Breastpump unit for expressing human breastmilk, wherein the breastpump unit has a breastshield (7) for placing on a human mother's breast and having an interior (70) for receiving the mother's breast, a vacuum pump (1) for generating an underpressure, and a suction line (4), wherein the suction line (4) connects the breastshield (7) to the vacuum pump (1) and wherein the suction line (4) transfers to the interior (70) of the breastshield (7) the underpressure generated by the vacuum pump (1), wherein the breastpump unit also has a pressure sensor (3), **characterized in that** a measuring line (6) is additionally present, and **in that** the measuring line (6) extends from the breastshield (7) to the pressure sensor (3) and that it serves to determine a pressure in the interior (70).

2. Breastpump unit according to Claim 1, wherein it has an electronic control unit (2) for controlling the vacuum pump (1), and wherein the pressure sensor (3) is connected to the electronic control unit (2).

3. Breastpump unit according to either of Claims 1 and 2, wherein the measuring line (6) and the suction line (4) are hoses.

4. Breastpump unit according to one of Claims 1 to 3, wherein the measuring line (6) and the suction line (4) are designed together as a double-lumen hose.

5. Breastpump unit according to one of Claims 1 to 4, wherein the suction line (4) has a first breastshield-side end (40), and the measuring line (6) has a second breastshield-side end (60), and wherein these two ends (40, 60) extend separately from each other.

6. Breastpump unit according to one of Claims 1 to 5, wherein the underpressure can be generated in a suction chamber (70) of the breastshield (7), and wherein the underpressure in the suction chamber can be measured by means of the measuring line.

7. Breastpump unit according to Claim 6, wherein the measuring line (6) opens into this suction chamber (70).

8. Breastpump unit according to one of Claims 1 to 6, wherein a protective element (8) is present, which separates the measuring line (6) from the interior (70) of the breastshield (7) into which expressed breastmilk can be admitted.

9. Breastpump unit according to one of Claims 1 to 6, wherein a media separation device (8) is present, which separates the measuring line (6) from the interior (70) of the breastshield (7).

10. Breastpump unit according to Claims 6 and 9, wherein the interior (70) is the suction chamber.

11. Breastpump unit according to Claims 8 and 9, wherein the protective element is the media separation device (8).

12. Breastpump unit according to one of Claims 9 to 11, wherein the media separation device is a flexible, fluid-impermeable membrane (8).

13. Breastpump unit according to one of Claims 1 to 12, wherein the measuring line (6) is releasably connectable to the breastshield (7) several times.

14. Breastpump unit according to one of Claims 1 to 13, wherein the measuring line (6) ends at the pressure sensor (3).

## Revendications

1. Unité de tire-lait pour pomper du lait maternel. humain, l'unité de tire-lait présentant une téterelle (7) destinée à être appliquée sur un sein maternel humain avec un espace interne (70) pour recevoir le sein maternel, une pompe à vide (1) pour générer une dépression et une conduite de succion (4), la conduite de succion (4) reliant la téterelle (7) à la pompe à vide (1) et la conduite de succion (4) transmettant la dépression générée par la pompe à vide (1) dans l'espace interne (70) de la téterelle (7), l'unité de tire-lait présentant en outre un capteur de pression (3), **caractérisée en ce qu'**il est prévu en outre une conduite de mesure (6) et **en ce que** la conduite de mesure (6) s'étend depuis la téterelle (7) jusqu'au capteur de pression (3) et sert à déterminer une pression dans l'espace interne (70).

2. Unité de tire-lait selon la revendication 1, présentant une commande électronique (2) pour commander la pompe à vide (1), le capteur de pression (3) étant connecté à la commande électronique (2).

3. Unité de tire-lait selon l'une quelconque des revendications 1 et 2, dans laquelle la conduite de mesure (6) et la conduite de succion (4) sont des tuyaux flexibles.

4. Unité de tire-lait selon l'une quelconque des revendications 1 à 3, dans laquelle la conduite de mesure (6) et la conduite de succion (4) sont réalisées en commun sous forme de tuyau souple à double lumière.

5. Unité de tire-lait selon l'une quelconque des revendications 1 à 4, dans laquelle la conduite de succion (4) présente une première extrémité (40) côté téterelle et la conduite de mesure (6) présente une deuxième extrémité (60) côté téterelle et dans laquelle ces deux extrémités (40, 60) s'étendent de manière séparée l'une de l'autre.

6. Unité de tire-lait selon l'une quelconque des revendications 1 à 5, dans laquelle la dépression peut être générée dans une chambre de succion (70) de la téterelle (7) et dans laquelle la dépression dans la chambre de succion peut être mesurée au moyen de la conduite de mesure.

7. Unité de tire-lait selon la revendication 6, dans laquelle la conduite de mesure (6) débouche dans cette chambre de succion (70).

8. Unité de tire-lait selon l'une quelconque des revendications 1 à 6, dans laquelle un élément de protection (8) est prévu, lequel sépare la conduite de mesure (6) de l'espace interne (70) de la téterelle (7) pouvant être sollicité avec le lait maternel pompé.

9. Unité de tire-lait selon l'une quelconque des revendications 1 à 6, dans laquelle un dispositif de séparation de milieux (8) est prévu, lequel sépare la conduite de mesure (6) de l'espace interne (70) de la téterelle (7).

10. Unité de tire-lait selon les revendications 6 et 9, dans laquelle l'espace interne (70) est la chambre de succion.

11. Unité de tire-lait selon les revendications 8 et 9, dans laquelle l'élément de protection est le dispositif de séparation de milieux (8).

12. Unité de tire-lait selon l'une quelconque des revendications 9 à 11, dans laquelle le dispositif de séparation de milieux est une membrane flexible (8) imperméable aux fluides.

13. Unité de tire-lait selon l'une quelconque des revendications 1 à 12, dans laquelle la conduite de mesure (6) peut être connectée plusieurs fois de manière amovible à la téterelle (7).

14. Unité de tire-lait selon l'une quelconque des revendications 1 à 13, dans laquelle la conduite de mesure (6) se termine au niveau du capteur de pression (3).
